(19) Europäisches Patentamt
European Patent Office
Office européen des brevets

(11) Veröffentlichungsnummer: **0 280 992**
**B1**

(12) # EUROPÄISCHE PATENTSCHRIFT

(45) Veröffentlichungstag der Patentschrift: **24.10.90**

(51) Int. Cl.[5]: **C07C 255/23**, C07C 69/757, C07C 67/00, C07C 317/12, A61K 31/275, A61K 31/215

(21) Anmeldenummer: **88102603.3**

(22) Anmeldetag: **23.02.88**

(54) **Cyclohexencarbonsäureester, Verfahren zu ihrer Herstellung und ihre Verwendung.**

(30) Priorität: **04.03.87 DE 3706877**

(43) Veröffentlichungstag der Anmeldung: **07.09.88 Patentblatt 88/36**

(45) Bekanntmachung des Hinweises auf die Patenterteilung: **24.10.90 Patentblatt 90/43**

(84) Benannte Vertragsstaaten: **AT BE CH DE ES FR GB IT LI NL SE**

(56) Entgegenhaltungen:
**DE-B-2 166 019**

**CHEMICAL ABSTRACTS, Band 82, Nr. 19, 12. Mai 1975, Columbus, Ohio, USA;J.S. BRIMACOMBE et al.: "Michael condensation of gamma-butyrolactones with cinnamate esters" Seite 543, Spalten 1, 2, Zusammenfassung Nr. 125196t**
**CHEMICAL ABSTRACTS, Band 98, Nr. 23, 6. Juni 1983, Columbus, Ohio, USA;N.S. PROSTAKOV et al.: "Cyano-ethylation of azafluorenes and syntheses of alcohols with azafluorene fragments" Seite 630, Spalten 1, 2, Zusammenfassung Nr. 197983a**

(73) Patentinhaber: **BAYER AG, D-5090 Leverkusen 1 Bayerwerk(DE)**

(72) Erfinder: **Meyer, Horst, Dr., Miles Pharmaceuticals 400 Morgan Lane, West Haven Connecticut(US)**
Erfinder: **Schwenner, Eckhard, Dr., Paul-Ehrlich-Strasse 29, D-5600 Wuppertal 1(DE)**
Erfinder: **Bechem, Martin, Dr., Obere Bergerheide 4, D-5600 Wuppertal 1(DE)**
Erfinder: **Gross, Rainer, Prof. Dr., Platzhofstrasse 23, D-5600 Wuppertal 1(DE)**
Erfinder: **Schramm, Matthias, Dr., Paffrather Strasse 38, D-5000 Köln 80(DE)**
Erfinder: **Kayser, Michael, Dr., Fleyerstrasse 231, D-5800 Hagen(DE)**
Erfinder: **Hebisch, Siegbert, Dr., Herzogstrasse 129, D-4200 Oberhausen 1(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft Cyclohexencarbonsäureester, ein Verfahren zu ihrer Herstellung sowie ihre Verwendung in Arzneimittel, insbesondere in kreislaufbeeinflussenden Arzneimitteln.

Die Erfindung betrifft Cyclohexencarbonsäureester der allgemeinen Formel (I)

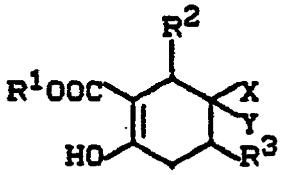

(I)

in welcher

$R^1$ - für einen geradkettigen, verweigten oder cyclischen, gesättigten oder ungesättigten Kohlenwasserstoffrest mit bis zu 20 Kohlenstoffatomen steht, der durch ein Sauerstoffatom oder ein Schwefelatom in der Kette unterbrochen sein kann und/oder der substituiert sein kann durch Halogen, Cyano, Hydroxy, Acetyloxy, Nitro, Nitrooxy oder durch eine gegebenenfalls durch Halogen, Cyano, Dialkylamino mit jeweils 1 bis 2 Kohlenstoffatomen je Alkylgruppe, Alkoxy mit 1 bis 4 Kohlenstoffatomen, Alkyl mit 1 bis 4 Kohlenstoffatomen, Trifluormethyl oder Nitro substituierte Phenyl-, Phenoxy-, Phenylthio- oder Phenylsulfonylgruppe, oder durch eine α-, β- oder γ-Pyridylgruppe oder durch eine Aminogruppe, wobei diese Aminogruppe 2 gleiche oder verschiedene Substituenten aus der Gruppe Alkyl mit bis zu 4 Kohlenstoffatomen, Alkoxyalkyl mit bis zu 4 Kohlenstoffatomen, Phenyl oder $C_7$-$C_{14}$-Aralkyl trägt, oder wobei diese Substituenten gegebenenfalls mit dem Stickstoffatom einer fünf- bis siebengliedrigen Ring bilden, der als weiteres Heteroatom ein Sauerstoff-oder Schwefelatom, oder eine N-Phenyl- oder N-Alkylgruppierung enthalten kann, wobei diese Alkylgruppe 1 bis 3 Kohlenstoffatome umfaßt,

$R^2$ - für $C_6$-$C_{12}$-Aryl steht, das bis zu 3-fach gleich oder verschieden durch Halogen, $C_1$-$C_8$-Alkyl, $C_1$-$C_8$-Alkoxy, $C_1$-$C_6$-Alkylthio, $C_1$-$C_6$-Alkylsulfonyl, Trifluormethyl, Trifluormethoxy, Difluormethoxy, Trifluormethylthio, Nitro, Cyano, $C_1$-$C_4$-Alkoxycarbonyl, Di-$C_1$-$C_6$-Alkylamino, Dimethylaminosulfonyl oder Dimethylcarbamoyl substituiert sein kann,

$R^3$ - für $C_1$-$C_6$-Alkyl oder

- für $C_6$-$C_{12}$-Aryl steht, das bis zu 2-fach gleich oder verschieden durch Halogen, $C_1$-$C_4$-Alkyl, $C_1$-$C_4$-Alkoxy, Cyano, Trifluormethyl oder Trifluormethoxy substituiert sein kann

und

X,Y- gleich oder verschieden sind und

- für eine Gruppe der Formel -CN, $-COR^4$, $-SO_2R^4$, $-COOR^5$, $-CONR^6R^7$ oder $-SO_2NR^6R^7$ stehen,

worin

$R^4$ - $C_1$-$C_8$-Alkyl, $C_6$-$C_{12}$-Aryl oder $C_7$-$C_{14}$-Aralkyl bedeutet,

$R^5$ - $C_1$-$C_8$-Alkyl oder $C_6$-$C_{12}$-Aryl bedeutet,

und

$R^6$,$R^7$ gleich oder verschieden sind und $C_1$-$C_6$-Alkyl, $C_6$-$C_{12}$-Aryl oder $C_7$-$C_4$-Aralkyl bedeuten,

in Form ihrer Isomeren, Isomerengemische, optischen Antipoden oder Racemate.

Bevorzugt seien Verbindungen der allgemeinen Formel (I) genannt,

in welcher

$R^1$ - für einen geradkettigen, verzweigten oder cyclischen, gesättigten oder ungesättigen Kohlenwasserstoffrest mit bis zu 14 Kohlenstoffatomen steht, der durch ein Sauerstoffatom in der Kette unterbrochen sein kann und/oder der substituiert sein kann durch Fluor, Chlor, Brom, Iod, Cyano, Hydroxy, Acetoxy oder durch ein gegebenenfalls durch Fluor, Chlor, Brom, Alkoxy mit 1 bis 4 Kohlenstoffatomen, Alkyl mit 1 bis 4 Kohlenstoffatomen oder Trifluormethyl substituierte Phenyl- oder Phenoxygruppe, oder durch eine α-, β-oder γ-Pyridylgruppe oder durch eine Aminogruppe, wobei diese Aminogruppe 2 gleiche oder verschiedene Substituenten aus der Gruppe Alkyl mit 1 bis 4 Kohlenstoffatomen, Phenyl oder Benzyl trägt,

$R^2$ - für Phenyl oder Naphthyl steht, wobei die genannten Reste bis zu 2-fach gleich oder verschieden durch Fluor, Chlor, Brom, $C_1$-$C_6$-Alkyl, $C_1$-$C_6$-Alkoxy, $C_1$-$C_4$-Alkylthio, $C_1$-$C_4$-Alkylsulfonyl, Trifluormethyl, Trifluormethoxy, Difluormethoxy, Nitro, Cyano, $C_1$-$C_4$-Alkoxycarbonyl oder Di-$C_1$-$C_4$-Alkylamino substiutiert sein können,

$R^3$ - für $C_1$-$C_4$-Alkyl oder

- für Phenyl oder Naphthyl steht, wobei die genannten Reste durch Fluor, Chlor, Methyl, Methoxy oder Trifluormethyl substituiert sein können

und

X,Y- gleich oder verschieden sind und

- für eine Gruppe der Formel -CN, $-COR^4$, $-SO_2R^4$, $-COOR^5$, $-CONR^6R^7$ oder $-SO_2NR^6R^7$ stehen,

worin

$R^4$ - $C_1$-$C_6$-Alkyl, Phenyl oder Benzyl bedeutet,
$R^5$ - $C_1$-$C_6$-Alkyl oder Phenyl bedeutet,
und
$R^6$,$R^7$ gleich oder verschieden sind und $C_1$-$C_4$-Alkyl, Phenyl oder Benzyl bedeuten,
in Form ihrer Isomeren, Isomerengemische, optischen Antipoden oder Racemate,

Besonders bevorzugt seien Verbindungen der allgemeinen Formel (I) genannt,
in welcher
$R^1$ - für einen geradkettigen, verzweigten oder cyclischen Kohlenwasserstoffrest mit bis zu 8 Kohlenstoffatomen steht, der durch ein Sauerstoffatom in der Kette unterbrochen sein kann und/oder der durch Fluor, Cyano, Acetoxy, Hydroxy, Phenyl, Phenoxy, α-, β- oder γ-Pyridyl oder durch eine Aminogruppe substituiert sein kann, wobei diese Aminogruppe zwei gleiche oder verschiedene Substituenten aus der Gruppe Alkyl mit 1 bis 2 Kohlenstoffatomen und Benzyl trägt,
$R^2$ - für Phenyl steht, das bis zu 2-fach gleich oder verschieden durch Fluor, Chlor, $C_1$-$C_4$-Alkyl, $C_1$-$C_4$-Alkoxy, Trifluormethyl oder Nitro substituiert sein kann,
$R^3$ - für Methyl oder Phenyl steht,
und
X,Y gleich oder verschieden sind und
- für eine Gruppe der Formel -CN, $-COR^4$, $-SO_2R^4$ oder $-COOR^5$ stehen,
worin
$R^4$ - $C_1$-$C_4$-Alkyl, Phenyl oder Benzyl bedeutet
und
$R^5$ - $C_1$-$C_4$-Alkyl bedeutet,
in Form ihrer Isomeren, Isomerengemische, optischen Antipoden oder Racemate.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Die erfindungsgemäßen Cyclohexencarbonsäureester der allgemeinen Formel (I) können hergestellt werden,
indem man
Acetessigester der allgemeinen Formel (II)

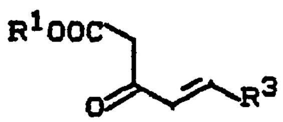

(II)

in welcher
$R^1$ und $R^3$ die oben agegebene Bedeutung haben,
mit Ethenen die allgemeinen Formel (III),

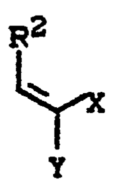

(III)

in welcher
$R^2$, X und Y die oben angegebene Bedeutung haben, in inerten Lösemitteln, in Anwesenheit von Basen umsetzt.

Verwendet man als Ausgangsstoffe 3-Oxo-5-phenyl-4-pentensäure-methylester und Benzylidenmalondinitril, so läßt sich das erfindungsgemäße Verfahren durch folgendes Schema verdeutlichen:

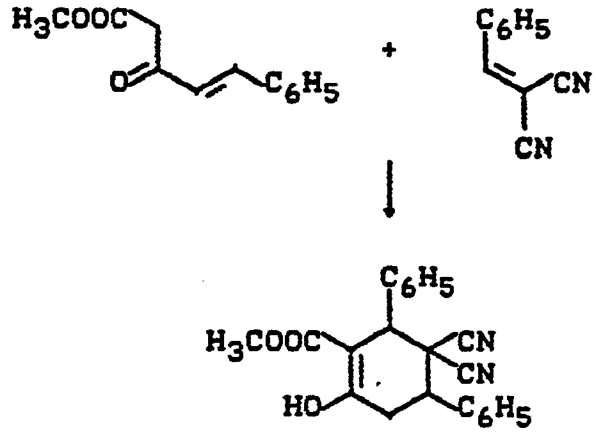

Als inerte Lösemittel eignen sich Wasser und/oder organische Lösemittel, die sich unter den Reaktionsbedingungen nicht verändern. Hierzu gehören bevorzugt Alkohole wie Methanol, Ethanol, Propanol, Isopropanol oder Butanol, oder Halogenkohlenwasserstoffe wie Methylenchlorid, Chloroform, Tetrachlorkohlenstoff, 1,2-Dichlorethan oder Dichlorethylen, oder Ether wie Diethylether, Butylmethylether, Dioxan oder Tetrahydrofuran, oder Kohlenwasserstoffe wie Benzol, Toluol oder Xylol, oder Amide wie Dimethylformamid oder Hexamethylphosphorsäuretriamid, oder Essigester, Dimethylsulfoxid, Sulfolan, Pyridin, Dimethylaminopyridin, Picolin, Morpholin oder Piperidin. Ebenso ist es möglich, Gemische der genannten Lösemittel einzusetzen.

Als Basen eignen sich die üblichen anorganischen oder organischen basischen Verbindungen. Hierzu gehören bevorzugt Alkali- oder Erdalkalihydroxide wie Natriumhydroxid, Kaliumhydroxid oder Bariumhydroxid, oder Alkalicarbonate wie Natriumcarbonat, Natriumhydrogencarbonat oder Kaliumcarbonat, oder Alkalialkoholate wie Natriummethanolat, Natriumethanolat, Kaliummethanolat, Kaliumethanolat oder Kaliumtert,butanolat, oder Ammoniak, oder organische Amine, die sich von Ammoniak ableiten, wie beispielsweise Triethylamin, Diisopropylamin, Ethyldiisopropylamin, oder organische Basen wie Pyridin, Dimethylaminopyridin, Picolin, Morpholin, Thiomorpholin, Piperidin, 1,5-Diazabicylco[4.3.0]non-5-en (DBN) oder 1,5-Diazabicyclo[5.4.0]undec-5-en (BDU).

Besonders bevorzugt wird die Umsetzung in Lösemitteln wie Wasser und/oder Alkoholen wie Methaol, Ethanol, Propanol oder Isopropanol mit wässriger Ammoniaklösung, oder Alkalialkoholaten durchgeführt.

Die Reaktion kann bei Normaldruck, bei erhöhtem oder bei erniedrigtem Druck durchgeführt werden. Im allgemeinen arbeitet man bei Normaldruck.

Die Reaktion wird im allgemeinen in einem Temperaturbereich von 0°C bis +100°C, bevorzugt von +20°C bis +80°C durchgeführt.

Bei der Durchführung der Reaktion wird im allgemeinen der Acetessigester in einer Menge von 0,5 bis 5, bevorzugt von 1 bis 2 Mol, bezogen auf 1 Mol des Ethens eingesetzt. Die Base wird im allgemeinen in einer Menge von 0.01 bis 5 Mol, bevorzugt von 0.05 bis 1 Mol, bezogen auf 1 Mol des Acetessigesters eingesetzt.

Die Umsetzung wird im allgemeinen derart durchgeführt, daß man den Acetessigester, das Ethen und Base in einem geeigneten Lösemittel mischt und gegebenenfalls erwärmt. Die Aufarbeitung erfolgt in üblicher Weise durch Extraktion, Chromatographie und/oder Kristallisation.

Die als Ausgangsstoffe eingesetzten Acetessigester sind bekannt oder können nach bekannten Methoden beispielsweise aus den entsprechenden Aldehyden ($R^3CHO$) mit den entsprechenden Phosphoranen ($R_3P{=}CHCOCH_2COOR^1$) bzw. Phosphonaten ($(RO_2)_2PO{-}CH_2{-}COCH_2{-}COOR^1$) durch Wittig- bzw. Wittig-Horner-Reaktion hergestellt werden [Houben-Weyl's "Methoden der organischen Chemie" V/1b; V/1c; E1; G. Wittig, U. Schöllkopf Org. Synth., Coll. Vol. V, 751 (1973)].

Die Ethene der allgemeinen Formel (III) sind bekannt oder können nach bekannten Methoden hergestellt werden [Beilstein's Handbuch der organischen Chemie 3 (1), 134; 9, 893].

Die erfindungsgemäßen Verbindungen zeigen ein nicht vorhersehbares, wertvolles pharmakologisches Wirkspektrum. Sie beeinflussen die Kontraktionskraft des Herzens und den Tonus der glatten Muskulatur. Sie können deshalb in Arzneimitteln zur Beeinflussung des pathologisch veränderten Blutdrucks, als Koronartherapeutika und zu Behandlung der Herzinsuffizienz, eingesetzt werden. Darüber hinaus können sie zur Behandlung von Herzrhythmusstörungen, zur Senkung des Blutzuckers, zur Abschwellung von Schleimhäuten und zur Beeinflussung des Salz- und Flüssigkeitshaushaltes verwendet werden.

Die Herzwirkung wurde an isolierten Vorhöfen von Meerschweinchenherzen gefunden.

Dazu werden die linken Verhöfe von Meerschweinchenherzen isoliert, und in ein auf 32°C thermostatisiertes Organbad gehängt. Als Inkubationsmedium dient eine Krebs-Henseleit-Lösung mit folgender Zusammensetzung (118,5 mmol/l NaCl, 4,75 mmol/l KCl, 1,19 mmol/l $KH_2PO_4$, 119 mmol/l $MgSO_4$, 25 mmol/l $NaHCO_3$, 0.013 mmol/l NaEDTA, 1,8 mmol/l $CaCl_2$) unter Zusatz von 10 mmol/l Glukose als energielieferndes Substrat. Die Lösung wird mit Carbogen (95% $CO_2$, 5% $O_2$) zur Aufrechterhaltung eines pH-Wertes von 7,4 begast. Die linken Vorhöfe werden unter Einstellung eines bestimmten Grundtonus in das Organbad eingespannt, und die Spannung mittels eines Kraftaufnehmers registriert. Unter periodischer elektrischer Reizung werden die dabei erfolgenden Kontraktionen fortlaufend auf einem Schnellschreiber aufgezeichnet. In Gegenwart der jeweiligen erfindungsgemäßen Verbindungen kommt es dabei zu einer prozentualen Verminderung der Kontraktionskraft gegenüber dem gleich 100% gesetzten Ausangswert:

| Bsp. | Konzentration (g/l) | %-Änderung der Kontraktionskraft |
|---|---|---|
| 6 | $10^{-3}$ | -43% |
| 16 | $10^{-3}$ | -27% |
| 18 | $10^{-3}$ | -25% |

Die neuen Wirkstoffe können in bekannter Weise in die üblicher Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90- Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.b: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständliche außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der

vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Herstellungsbeispiele

Beispiel 1

3-Acetyl-2-(2-ethoxyphenyl)-6-hydroxy-4-phenyl-cylcohex-6-en-1,3-dicarbonsäure-diethylester

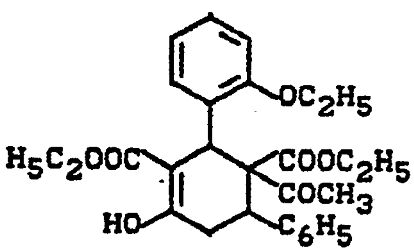

Eine Lösung von 2,6 g (0,01 mol) 2-Acetyl-3-(2-ethoxyphenyl)propensäureethylester in 20 ml Ethanol wird nacheinander mit 1 ml 1%iger Natriumethanolat/Ethanol-Lösung und einer Lösung von 2,2 g (0,01 mol) 3-Oxo-5-phenyl-4-pentensäure-ethylester in 10 ml Ethanol versetzt und 1 d bei Raumtemperatur gerührt. Danach wird abgesaugt und der Rückstand mit Ethanol gewaschen.
Ausbeute: 2,9 g (60,3% der Theorie)
Schmp.: 162°C

Beispiel 2

3-Cyano-6-hydroxy-3-methylsulfonyl-2-(2-nitrophenyl)-4-phenyl-cyclohex-6-en-1-carbonsäure-ethylester

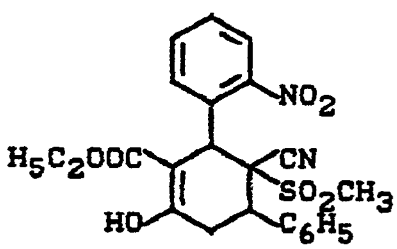

Eine Mischung aus 2,2 g (0,01 mol) 3-Oxo-5-phenyl-4-pentensäure-ethylester, 2,5 g (0,01 mol) 1-Methylsulfonyl-2-(2-nitrophenyl)-acrylnitril und 1,1 ml konz. Ammoniak in 15 ml Ethanol wird 2 Tage bei Raumtemperatur gerührt. Danach wird abgesaugt und der Rückstand mit Ethanol gewaschen.
Ausbeute: 2,7 g (57,3% der Theorie)
Schmp.: 180°C

Analog den Beispielen 1 und 2 wurden die in der folgenden Tabelle aufgeführten Verbindungen synthetisiert:

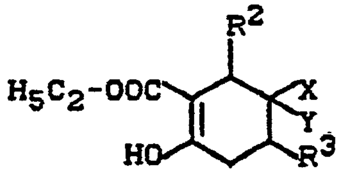

| Bsp.-Nr. | $R^2$ | $R^3$ | X | Y | Fp. [°C] | Ausbeute [% d.Th. |
|---|---|---|---|---|---|---|
| 3 | 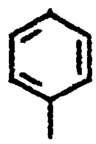 | $C_6H_5$ | CN | CN | 187 | 88,7 |

| Bsp.-Nr. | $R^2$ | $R^3$ | X | Y | Fp. [°C] | Ausbeute [% d.Th.] |
|---|---|---|---|---|---|---|
| 4 | 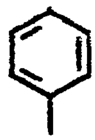 | $C_6H_5$ | $COOC_2H_5$ | CN | 128 | 83,4 |
| 5 | 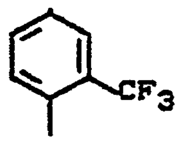 | $C_6H_5$ | $COOC_2H_5$ | CN | 124 | 65,8 |
| 6 | 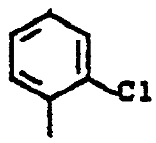 | $C_6H_5$ | $COOC_2H_5$ | CN | 161 | 46,3 |
| 7 | 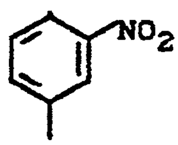 | $C_6H_5$ | $COOC_2H_5$ | CN | 134 | 43,1 |
| 8 | 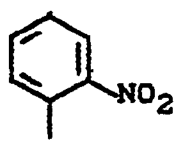 | $C_6H_5$ | $COOC_2H_5$ | CN | 206 | 90,5 |
| 9 | 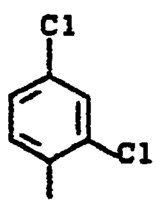 | $C_6H_5$ | $COOC_2H_5$ | CN | 136 | 59,0 |
| 10 | 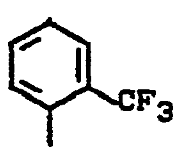 | $C_6H_5$ | $SO_2CH_3$ | CN | 201 | 28,4 |

| Bsp.-Nr. | $R^2$ | $R^3$ | X | Y | Fp. [°C] | Ausbeute [% d.Th.] |
|---|---|---|---|---|---|---|
| 11 | 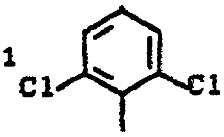 | $C_6H_5$ | $COOCH_3$ | CN | 125 | 52,7 |
| 12 | 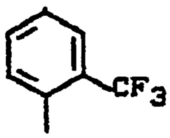 | $C_6H_5$ | $COOC_2H_5$ | $COOC_2H_5$ | 102 | 20,6 |
| 13 | 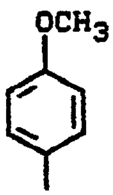 | $C_6H_5$ | $COOC_2H_5$ | CN | 157 | 15,6 |
| 14 | 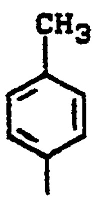 | $C_6H_5$ | $COOC_2H_5$ | CN | 184 | 69,3 |
| 15 | 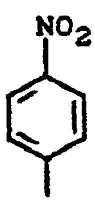 | $C_6H_5$ | $COOC_2H_5$ | CN | 182 | 73,3 |
| 16 | 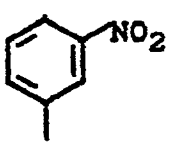 | $C_6H_5$ | $COOC_2H_5$ | $COOC_2H_5$ | 116 | 45,0 |
| 17 | 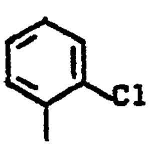 | $C_6H_5$ | $SO_2CH_3$ | CN | 179 | 65,3 |

| Bsp.-Nr. | $R^2$ | $R^3$ | X | Y | Fp. [°C] | Ausbeute [% d.Th.] |
|---|---|---|---|---|---|---|
| 18 | 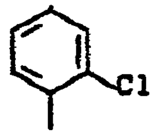 | $C_6H_5$ | $COOC_2H_5$ | $COCH_3$ | 132 | 74,5 |
| 19 | 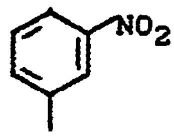 | $CH_3$ | $COOC_2H_5$ | $COOC_2H_5$ | 105 | 22,3 |
| 20 | 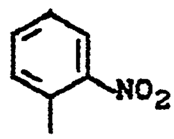 | $C_6H_5$ | $COOC_2H_5$ | $COOC_2H_5$ | 149 | 15,7 |
| 21 | 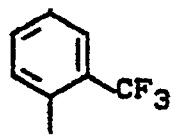 | $C_6H_5$ | $COOC_2H_5$ | $COCH_3$ | 144 | 19,8 |
| 22 | 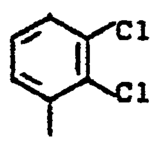 | $C_6H_5$ | $COOC_2H_5$ | $COCH_3$ | 120 | 19,5 |
| 23 | 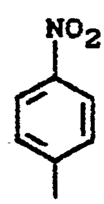 | $CH_3$ | $COOC_2H_5$ | CN | 102 | 19,9 |
| 24 | 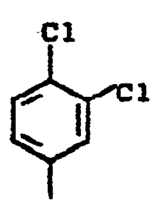 | $C_6H_5$ | $COOC_2H_5$ | $COCH_3$ | 178 | 25,7 |

## Patentansprüche

1. Cyclohexencarbonsäuren der allgemeinen Formel (I)

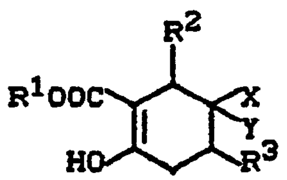

(I)

in welcher

$R^1$ - für einen geradkettigen, verzweigten oder cyclischen, gesättigten oder ungesättigten Kohlenwasserstoffrest mit bis zu 20 Kohlenstoffatomen steht, der durch ein Sauerstoffatom oder ein Schwefelatom in der Kette unterbrochen sein kann und/oder der substituiert sein kann durch Halogen, Cyano, Hydroxy, Acetyloxy, Nitro oder durch eine gegebenenfalls durch Halogen, Cyano, Dialkylamino mit jeweils 1 bis 2 Kohlenstoffatomen je Alkylgruppe, Alkoxy mit 1 bis 4 Kohlenstoffatomen, Alkyl mit 1 bis 4 Kohlenstoffatomen, Trifluormethyl oder Nitro substituierte Phenyl-, Phenoxy-, Phenylthio- oder Phenylsulfonylgruppe, oder durch eine α-, β-oder γ-Pyridylgruppe oder durch eine Aminogruppe, wobie diese Aminogruppe 2 gleiche oder verschiedene Substituenten aus der Gruppe Alkyl mit bis zu 4 Kohlenstoffatomen, Alkoxyalkyl mit bis zu 4 Kohlenstoffatomen, Phenyl oder $C_7$-$C_{14}$-Aralkyl trägt, oder wobei diese Substituenten gegebenenfalls mit dem Stickstoffatom einen fünf-bis siebengliedrigen Ring bilden, der als weiteres Heteroatom ein Sauerstoff-oder Schwefelatom, oder eine N-Phenyl- oder N-Alkylgruppierung enthalten kann, wobei diese Alkylgruppe 1 bis 3 Kohlenstoffatome umfaßt,

$R^2$ - für $C_6$-$C_{12}$-Aryl steht, das bis zu 3-fach gleich oder verschieden durch Halogen, $C_1$-$C_8$-Alkyl, $C_1$-$C_8$-Alkoxy, $C_1$-$C_6$-Alkylthio, $C_1$-$C_6$-Alkylsulfonyl, Trifluormethyl, Trifluormethoxy, Difluormethoxy, Trifluormethylthio, Nitro, Cyano, $C_1$-$C_4$-Alkoxycarbonyl, Di-$C_1$-$C_6$-Alkylamino, Dimethylaminosulfonyl oder Dimethylcarbamoyl substituiert sein kann,

$R^3$ - für $C_1$-$C_6$-Alkyl oder

- für $C_6$-$C_{12}$-Aryl steht, das bis zu 2-fach gleich oder verschieden durch Halogen, $C_1$-$C_4$-Alkyl, $C_1$-$C_4$-Alkoxy, Cyano, Trifluormethyl oder Trifluormethoxy substituiert sein kann

und

X,Y- gleich oder verschieden sind und

- für eine Gruppe der Formel -CN, $-COR^4$, $-SO_2R^4$, $-COOR^5$, $-CONR^6R^7$ oder $-SO_2NR^6R^7$ stehen,

worin

$R^4$ - $C_1$-$C_8$-Alkyl, $C_6$-$C_{12}$-Aryl oder $C_7$-$C_{14}$-Aralkyl bedeutet,

$R^5$ - $C_1$-$C_8$-Alkyl oder $C_6$-$C_{12}$-Aryl bedeutet,

und

$R^6$,$R^7$ gleich oder verschieden sind und $C_1$-$C_6$-Alkyl, $C_6$-$C_{12}$-Aryl oder $C_7$-$C_{14}$-Aralkyl bedeuten,

in Form ihrer Isomeren, Isomerengemische, optischen Antipoden oder Racemate.

2. Verbindungen der allgemeinen Formel (I) gemäß Anspruch 1, in welcher

$R^1$ - für einen geradkettigen, verzweigten oder cyclischen, gesättigten oder ungesättigen Kohlenwasserstoffrest mit bis zu 14 Kohlenstoffatomen steht, der durch ein Sauerstoffatom in der Kette unterbrochen sein kann und/oder der substituiert sein kann durch Fluor, Chlor, Brom, Iod, Cyano, Hydroxy, Acetoxy oder durch eine gegebenenfalls durch Fluor, Chlor, Brom, Alkoxy mit 1 bis 4 Kohlenstoffatomen, Alkyl mit 1 bis 4 Kohlenstoffatomen oder Trifluormethyl substituierte Phenyl- oder Phenoxygruppe, oder durch eine α-, β-oder γ-Pyridylgruppe oder durch eine Aminogruppe, wobei diese Aminogruppe 2 gleiche oder verschiedene Substituenten aus der Gruppe Alkyl mit 1 bis 4 Kohlenstoffatomen, Phenyl oder Benzyl trägt,

$R^2$ - für Phenyl oder Naphthyl steht, wobei die genannten Reste bis zu 2-fach gleich oder verschieden durch Fluor, Chlor, Brom, $C_1$-$C_6$-Alkyl, $C_1$-$C_6$-Alkoxy, $C_1$-$C_4$-Alkylthio, $C_1$-$C_4$-Alkylsulfonyl, Trifluormethyl, Trifluormethoxy, Difluormethoxy, Nitro, Cyano, $C_1$-$C_4$-Alkoxycarbonyl oder Di-$C_1$-$C_4$-Alkylamino substituiert sein können,

$R^3$ - für $C_1$-$C_4$-Alkyl oder

- für Phenyl oder Naphthyl steht, wobei die genannten Reste durch Fluor, Chlor, Methyl, Methoxy oder Trifluormethyl substituiert sein können

und

X,Y- gleich oder verschieden sind und

- für eine Gruppe der Formel -CN, $-COR^4$, $-SO_2R^4$, $-COOR^5$, $-CONR^6R^7$ oder $-SO_2NR^6R^7$ stehen,

worin

$R^4$ - $C_1$-$C_6$-Alkyl, Phenyl oder Benzyl bedeutet,

$R^5$ - $C_1$-$C_6$-Alkyl oder Phenyl bedeutet,

und

$R^6$,$R^7$ gleich oder verschieden sind und $C_1$-$C_4$-Alkyl, Phenyl oder Benzyl bedeuten, in Form ihrer Isomeren, Isomerengemische, optischen Antipoden oder Racemate.

3. Verbindungen der allgemeinen Formel (I) gemäß Anspruch 1, in welcher

$R^1$ - für einen geradkettigen, verzweigten oder cyclischen Kohlenwasserstoffrest mit bis zu 8 Kohlenstoffatomen steht, der durch ein Sauerstoffatom in der Kette unterbrochen sein kann und/oder der durch Fluor, Cyano, Acetoxy, Hydroxy, Phenyl, Phenoxy, α-, β- oder γ- Pyridyl oder durch eine Aminogruppe substituiert sein kann, wobei diese Aminogruppe zwei gleiche oder verschiedene Substituenten aus der Gruppe Alkyl mit 1 bis 2 Kohlenstoffatomen und Benzyl trägt,

$R^2$ - für Phenyl steht, das bis zu 2-fach gleich oder verschieden durch Fluor, Chlor, $C_1$-$C_4$-Alkyl, $C_1$-$C_4$-Alkoxy, Trifluormethyl oder Nitro substituiert sein kann,

$R^3$ - für Methyl oder Phenyl steht,

und

X,Y gleich oder verschieden sind und

- für eine Gruppe der Formel -CN, $-COR^4$, $-SO_2R^4$ oder $-COOR^5$ stehen,

worin

$R^4$ - $C_1$-$C_4$-Alkyl, Phenyl oder Benzyl bedeutet

und

$R^5$ - $C_1$-$C_4$-Alkyl bedeutet,

in Form ihrer Isomeren, Isomerengemische, optischen Antipoden oder Racemate.

4. Verfahren zur Herstellung von Cyclohexencarbonsäureestern der allgemeinen Formel (I)

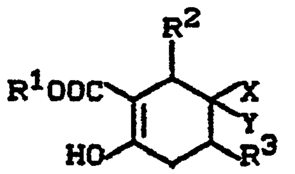

(I)

in welcher

$R^1$ - für einen geradkettigen, verzweigten oder cyclischen, gesättigten oder ungesättigten Kohlenwasserstoffrest mit bis zu 20 Kohlenstoffatomen steht, der durch ein Sauerstoffatom oder ein Schwefelatom in der Kette unterbrochen sein kann und/oder der substituiert sein kann durch Halogen, Cyano, Hydroxy, Acetyloxy, Nitro oder durch eine gegebenenfalls durch Halogen, Cyano, Dialkylamino mit jeweils 1 bis 2 Kohlenstoffatomen je Alkylgruppe, Alkoxy mit 1 bis 4 Kohlenstoffatomen, Alkyl mit 1 bis 4 Kohlenstoffatomen, Trifluormethyl oder Nitro substituierte Phenyl-, Phenoxy-, Phenylthio- oder Phenylsulfonylgruppe, oder durch eine α-, β- oder γ-Pyridylgruppe oder durch eine Aminogruppe, wobei diese Aminogruppe 2 gleiche oder verschiedene Substituenten aus der Gruppe Alkyl mit bis zu 4 Kohlenstoffatomen, Alkoxyalkyl mit bis zu 4 Kohlenstoffatomen, Phenyl oder $C_7$-$C_{14}$-Aralkyl trägt, oder wobei diese Substituenten gegebenenfalls mit dem Stickstoffatom einen fünf-bis siebengliedrigen Ring bilden, der als weiteres Heteroatom ein Sauerstoff-oder Schwefelatom, oder eine N-Phenyl- oder N-Alkylgruppierung enthalten kann, wobei diese Alkylgruppe 1 bis 3 Kohlenstoffatome umfaßt,

$R^2$ - für $C_6$-$C_{12}$-Aryl steht, das bis zu 3-fach gleich oder verschieden durch Halogen, $C_1$-$C_8$-Alkyl, $C_1$-$C_8$-Alkoxy, $C_1$-$C_6$-Alkylthio, $C_1$-$C_6$-Alkylsulfonyl, Trifluormethyl, Trifluormethoxy, Difluormethoxy, Trifluormethylthio, Nitro, Cyano, $C_1$-$C_4$-Alkoxycarbonyl, Di-$C_1$-$C_6$-Alkylamino, Dimethylaminosulfonyl oder Dimethylcarbamoyl substituiert sein kann,

$R^3$ - für $C_1$-$C_6$-Alkyl oder

- für $C_6$-$C_{12}$-Aryl steht, das bis zu 2-fach gleich oder verschieden durch Halogen, $C_1$-$C_4$-Alkyl, $C_1$-$C_4$-Alkoxy, Cyano, Trifluormethyl oder Trifluormethoxy substituiert sein kann

und

X,Y- gleich oder verschieden sind und

- für eine Gruppe der Formel -CN, $-COR^4$, $-SO_2R^4$, $-COOR^5$, $-CONR^6R^7$ oder $-SO_2NR^6R^7$ stehen,

worin

$R^4$ - $C_1$-$C_8$-Alkyl, $C_6C_{12}$-Aryl oder $C_7$-$C_{14}$-Aralkyl bedeutet,

$R^5$ - $C_1$-$C_8$-Alkyl oder $C_6$-$C_{12}$-Aryl bedeutet,

und

$R^6$,$R^7$ gleich oder verschieden sind und $C_1$-$C_6$-Alkyl, $C_6$-$C_{12}$-Aryl oder $C_7$-$C_{14}$-Aralkyl bedeuten,

dadurch gekennzeichnet, daß man Acetessigester der allgemeinen Formel (II)

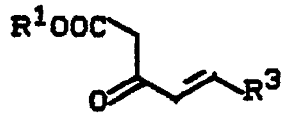

(II)

in welcher
$R^1$ und $R^3$ die oben angegebene Bedeutung haben,
mit Ethenen der allgemeinen Formel (II),

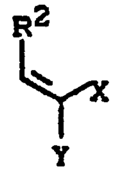

(III)

in welcher
$R^2$, X und Y die oben angegebene Bedeutung haben,
in Gegenwart von inerten Lösungsmittel, in Anwesenheit von Basen bei Temperaturen zwischen 0°C und 100°C umsetzt und gegebenenfalls ihre stereoisomeren Formen nach üblichen Methoden trennt.

5. Verfahren gemäß Anspruch 4, dadurch gekennzeichnet, daß man als inerte Lösungsmittel Wasser oder organische Lösungsmittel wie Alkohole, Halogenkohlenwasserstoffe, Ether, Kohlenwasserstoffe, Amide, Essigester, Dimethylsulfoxid, Sulfolan, Pyridin, Dimethylaminopyridin, Picolin, Morpholin oder Piperidin sowie Gemische hiervon einsetzt und als Basen Alkali- oder Erdalkalihydroxide, Carbonate, Alkoholate, Ammoniak oder organische Amine einsetzt.

6. Verbindungen der allgemeinen Formel (I) gemäß Anspruch 1 zur Verwendung bei der Bekämpfung von Herzkreislauferkrankungen.

7. Arzneimittel enthaltend mindestens eine Verbindung der allgemeinen Formel (I) gemäß Anspruch 1.

8. Verfahren zur Herstellung von Arzneimitteln, dadurch gekennzeichnet, daß man mindestens eine Verbindung der allgemeinen Formel (I) gemäß Anspruch 1 gegebenenfalls mit üblichen Hilfs- und Trägerstoffen in eine geeignete Applikationsform überführt.

9. Verwendung von Verbindungen der allgemeinen Formel (I) gemäß Anspruch 1 bei der Herstellung von kreislaufwirksamen Arzneimitteln.

**Claims**

. Cyclohexenecarboxylic acids of the general formula (I)

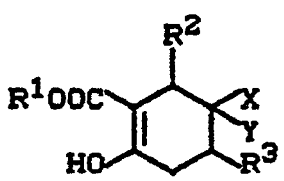

(I)

in which
$R^1$ represents a straight-chain or branched, or cyclic, saturated or unsaturated hydrocarbon radical with up to 20 carbon atoms, which can be interrupted by an oxygen atom or a sulphur atom in the chain and/or which can be substituted by halogen, cyano, hydroxyl, acetyloxy, nitro, or by a phenyl, phenoxy, phenylthio or phenylsulphonyl group which is optionally substituted by halogen, cyano, dialkylamino with in each case 1 to 2 carbon atoms to each alkyl group, alkoxy with 1 to 4 carbon atoms, alkyl with 1 to 4 carbon atoms, trifluoromethyl or nitro, or by an α-, β- or γ-pyridyl group or by an amino group, where this amino group carries 2 identical or different substituents from the group alkyl with up to 4 carbon atoms, alkoxyalkyl with up to 4 carbon atoms, phenyl or $C_7$–$C_{14}$-aralkyl, or where these substituents optionally form with the nitrogen atom a five- to seven-membered ring, which can contain an oxygen or sulphur atom as an additional hetero atom, or an N-phenyl or N-alkyl grouping, where this alkyl group comprises 1 to 3 carbon atoms,
$R^2$ represents $C_6$–$C_{12}$-aryl, which can be mono-, di- or trisubstituted by halogen, $C_1$–$C_8$-alkyl, $C_1$–$C_8$-alkoxy, $C_1$–$C_6$-alkylthio, $C_1$–$C_6$-alkylsulphonyl, trifluoromethyl, trifluoromethoxy, difluoromethoxy, trifluoromethylthio, nitro, cyano, $C_1$–$C_4$-alkoxycarbonyl, di-$C_1$–$C_6$-alkylamino, dimethylaminosulphonyl or dimethylcarbamoyl, the substituents being identical or different,

$R^3$ represents $C_1$–$C_6$-alkyl or represents $C_6$–$C_{12}$-aryl, which can be mono- or disubstituted by halogen, $C_1$–$C_4$-alkyl, $C_1$–$C_4$-alkoxy, cyano, trifluoromethyl or trifluoromethoxy, the substituents being identical or different, and

X and Y are identical or different and represent a group of the formula –CN, –$COR^4$, $SO_2R^4$, –CO-$OR^5$, –$CONR^6R^7$ or –$SO_2NR^6R^7$,

wherein

$R^4$ denotes $C_1$–$C_8$-alkyl, $C_6$–$C_{12}$-aryll or $C_7$–$C_{14}$-aralkyl,

$R^5$ denotes $C_1$–$C_8$-alkyl or $C_6$–$C_{12}$-aryl, and

$R^6$ and $R^7$ are identical or different and denote

$C_1$–$C_6$-alkyl, $C_6$–$C_{12}$-aryl or $C_7$–$C_{14}$-aralkyl, in the form of their isomers, isomeric mixtures, optical antipodes or racemates.

2. Compounds of the general formula (I) according to Claim 1, in which

$R^1$ represents a straight-chain or branched, or cyclic, saturated or unsaturated hydrocarbon radical with up to 14 carbon atoms, which can be interrupted by an oxygen atom in the chain and/or can be substituted by fluorine, chlorine, bromine, iodine, cyano, hydroxyl, acetoxy, or by a phenyl or phenoxy group which is optionally substituted by fluorine, chlorine, bromine, alkoxy with 1 to 4 carbon atoms, alkyl with 1 to 4 carbon atoms or trifluoromethyl, or by an α-, β- or γ-pyridyl group or by an amino group, where this amino group carries 2 identical or different substituents from the group alkyl with 1 to 4 carbon atoms, phenyl or benzyl,

$R^2$ represents phenyl or naphthyl, where the radicals mentioned can be mono- or disubstituted by fluorine, chlorine, bromine, $C_1$–$C_6$–alkyl, $C_1$–$C_6$–alkoxy, $C_1$–$C_4$-alkylthio, $C_1$–$C_4$–alkylsulphonyl, trifluoromethyl, trifluoromethoxy, difluoromethoxy, nitro, cyano, $C_1$–$C_4$–alkoxycarbonyl or di-$C_1$–$C_4$–alkylamino, the substituents being identical or different,

$R^3$ represents $C_1$–$C_4$–alkyl or represents phenyl or naphthyl, where the radicals mentioned can be substituted by fluorine, chlorine, methyl, methoxy or trifluoromethyl

and

X and Y are identical or different and represent a group of the formula –CN, –$COR^4$, –$SO_2R^4$, –CO-$OR^5$, –$CONR^6R^7$ or –$SO_2NR^6R^7$,

wherein

$R^4$ denotes $C_1$–$C_6$–alkyl, phenyl or benzyl, $R^5$ denotes $C_1$–$C_6$-alkyl or phenyl,

and

$R^6$ and $R^7$ are identical or different and denote $C_1$–$C_4$–alkyl, phenyl or benzyl,

in the form of their isomers, isomeric mixtures, optical antipodes or racemates.

3. Compounds of the general formula (I) according to Claim 1, in which

$R^1$ represents a straight-chain or branched or cyclic hydrocarbon radical with up to 8 carbon atoms, which can be interrupted by an oxygen atom in the chain and/or which can be substituted by fluorine, cyano, acetoxy, hydroxyl, phenyl, phenoxyl, α-, β- or γ-pyridyl or an amino group, where this amino group carries two identical or different substituents from the group alkyl with 1 to 2 carbon atoms and benzyl,

$R^2$ represents phenyl, which can be mono- or disubstituted by fluorine, chlorine, $C_1$–$C_4$–alkyl, $C_1$–$C_4$–alkoxy, trifluoromethyl or nitro, the substituents being identical or different,

$R^3$ represents methyl or phenyl, and

X and Y are identical or different and represent a group of the formula –CN, –CO $R^4$, –$SO_2R^4$ or –$COOR^5$,

wherein

$R^4$ denotes $C_1$–$C_4$-alkyl, phenyl or benzyl

and

$R^5$ denotes $C_1$–$C_4$-alkyl,

in the form of their isomers, isomeric mixtures, optical antipodes or racemates.

4. Process for the preparation of cyclohexenecarboxylates of the general formula (I)

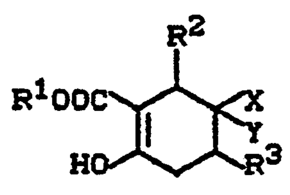

(I)

in which

$R^1$ represents a straight-chain or branched, or cyclic, saturated or unsaturated hydrocarbon radical with up to 20 carbon atoms, which can be interrupted by an oxygen atom or a sulphur atom in the chain and/or which can be substituted by halogen, cyano, hydroxyl, acetyloxy, nitro, or by a phenyl, phenoxy, phenylthio or phenylsulphonyl group which is optionally substituted by halogen, cyano, dialkylamino with in each case 1 to 2 carbon atoms to each alkyl group, alkoxy with 1 to 4 carbon atoms, alkyl with 1 to 4 carbon atoms, trifluoromethyl or nitro, or by an α-, β- or γ-pyridyl group or by an amino group, where this

amino group carries 2 identical or different substituents from the group alkyl with up to 4 carbon atoms, alkoxyalkyl with up to 4 carbon atoms, phenyl or $C_7$–$C_{14}$–aralkyl, or where these substituents optionally form with the nitrogen atom a five- to seven-membered ring, which can contain an oxygen or sulphur atom as an additional hetero atom, or an N-phenyl or N-alkyl grouping, where this alkyl group comprises 1 to 3 carbon atoms,

$R^2$ represents $C_6$–$C_{12}$-aryl, which can be mono-, di- or trisubstituted by halogen, $C_1$–$C_8$-alkyl, $C_1$–$C_8$-alkoxy, $C_1$–$C_6$-alkylthio, $C_1$–$C_6$-alkylsulphonyl, trifluoromethyl trifluoromethoxy, difluoromethoxy, trifluoromethylthio, nitro, cyano, $C_1$–$C_4$-alkoxycarbonyl, di-$C_1$–$C_6$-alkylamino, dimethylaminosulphonyl or dimethylcarbamoyl, the substituents being identical or different,

$R^3$ represents $C_1$–$C_6$-alkyl or represents $C_6$–$C_{12}$-aryl, which can be mono- or disubstituted by halogen, $C_1$–$C_4$-alkyl, $C_1$–$C_4$-alkoxy, cyano, trifluoromethyl or trifluoromethoxy, the substituents being identical or different,

and

X and Y are identical or different and represent a group of the formula $-CN$, $-COR^4$, $-SO_2R^4$, $-COOR^5$, $-CONR^6R^7$ or $-SO_2NR^6R^7$,

wherein

$R^4$ denotes $C_1$–$C_8$-alkyl, $C_6$–$C_{12}$-aryl or $C_7$–$C_{14}$-aralkyl,

$R^5$ denotes $C_1$–$C_8$-alkyl or $C_6$–$C_{12}$-aryl, and

$R^6$ and $R^7$ are identical or different and denote $C_1$–$C_6$-alkyl, $C_6$–$C_{12}$-aryl or $C_7$–$C_{14}$-aralkyl,

characterized in that acetoacetic esters of the general formula (II)

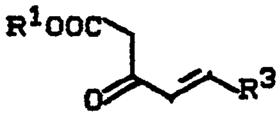

(II)

in which

$R^1$ and $R^3$ have the meaning given above, are reacted with ethenes of the general formula (III)

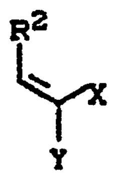

(III)

in which

$R^2$, X and Y have the meaning given above, in the presence of inert solvents and in the presence of bases at temperatures between 0°C and 100°C and are optionally separated into their stereoisomeric forms according to usual methods.

5. Process according to Claim 4, characterized in that water or organic solvents such as alcohols, halogenohydrocarbons, ethers, hydrocarbons, amides, ethyl acetate, dimethyl sulphoxide, sulpholane, pyridine, dimethylaminopyridine, picoline, morpholine or piperidine and also mixtures thereof are employed as inert solvents and alkali metal hydroxides or alkaline earth metal hydroxides, carbonates, alcoholates, ammonia or organic amines are employed as bases.

6. Compounds of the general formula (I) according to Claim 1 for use in the control of circulatory diseases.

7. Drugs containing at least one compound of the general formula (I) according to Claim 1.

8. Process for the preparation of drugs, characterized in that at least one compound of the general formula (I) according to Claim 1 is converted, optionally with the usual auxiliaries and excipients, into a suitable form for administration.

9. Use of compounds of the general formula (I) according to Claim 1 in the preparation of drugs which act on the circulatory system.

## Revendications

1. Acides cyclohexènecarboxyliques de formule générale (I)

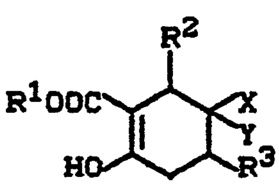

(I)

dans laquelle

$R^1$ représente un reste hydrocarboné saturé ou non saturé, à chaîne droite, à chaîne ramifiée ou cyclique ayant jusqu'à 20 atomes de carbone, qui peut être interrompu dans sa chaîne par un atome d'oxygène ou par un atome de soufre et/ou qui peut être substitué par un halogène, un radical cyano, hydroxy, acétyloxy, nitro ou par un groupe phényle, phénoxy, phénylthio ou phénylsulfonyle éventuellement substitué par un radical halogéno, cyano, dialkylamino ayant 1 ou 2 atomes de carbone par groupe alkyle, un radical alkoxy ayant 1 à 4 atomes de carbone, alkyle ayant 1 à 4 atomes de carbone, trifluorométhyle ou nitro, ou par un groupe α-, β- ou γ-pyridyle ou par un groupe amino, ce groupe amino portant 2 substituants, identiques ou différents, du groupe des substituants alkyle ayant jusqu'à 4 atomes de carbone, alkoxyalkyle ayant jusqu'à 4 atomes de carbone, phényle ou aralkyle en $C_7$ à $C_{14}$, ou bien ces substituants forment le cas échéant avec l'atome d'azote un noyau pentagonal à heptagonal qui peut comporter comme autre hétéroatome un atome d'oxygène ou de soufre ou un groupement N-phényle ou N-alkyle, ce groupe alkyle comprenant 1 à 3 atomes de carbone,

$R^2$ est un groupe aryle en $C_6$ à $C_{12}$, qui peut porter 1 à 3 substituants, identiques ou différents, halogéno, alkyle en $C_1$ à $C_8$, alkoxy en $C_1$ à $C_8$, alkylthio en $C_1$ à $C_6$, alkylsulfonyle en $C_1$ à $C_6$, trifluorométhyle, trifluorométhoxy, difluorométhoxy, trifluorométhylthio, nitro, cyano, (alkoxy en $C_1$ à $C_4$)carbonyle, di(alkyle en $C_1$ à $C_6$)amino, diméthylaminosulfonyle ou diméthylcarbamoyle,

$R^3$ est un groupe alkyle en $C_1$ à $C_6$ ou

un groupe aryle en $C_6$ à $C_{12}$, qui peut porter jusqu'à 2 substituants, identiques ou différents, halogéno, alkyle en $C_1$ à $C_4$, alkoxy en $C_1$ à $C_4$, cyano, trifluorométhyle ou trifluorométhoxy

et

X, Y – sont identiques ou différents et représentent

un groupe de formule $-CN$, $-COR^4$, $-SO_2R^4$, $-COOR^5$, $-CONR^6R^7$ ou $-SO_2NR^6R^7$, ou

$R^4$ est un radical alkyle en $C_1$ à $C_8$, aryle en $C_6$ à $C_{12}$ ou aralkyle en $C_7$ à $C_{14}$,

$R^5$ est un radical alkyle en $C_1$ à $C_8$ ou aryle en $C_6$ à $C_{12}$

et

$R^6$, $R^7$ sont identiques ou différents et représentent un radical alkyle en $C_1$ à $C_6$, aryle en $C_6$ à $C_{12}$ ou aralkyle en $C_7$ à $C_{14}$,

sous forme de leurs isomères, de leurs mélanges d'isomères, de leurs antipodes optiques ou de leurs racémates.

2. Composés de formule générale (I) suivant la revendication 1, dans laquelle

$R^1$ est un reste hydrocarboné saturé ou non saturé à chaîne droite, à chaîne ramifiée ou cyclique ayant jusqu'à 14 atomes de carbone, qui peut être interrompu dans sa chaîne par un atome d'oxygène et/ou qui peut être substitué par du fluor, du chlore, du brome, de l'iode, un radical cyano, hydroxy, acétoxy ou par un groupe phényle ou phénoxy éventuellement substitué par du fluor, du chlore, du brome, un radical alkoxy ayant 1 à 4 atomes de carbone, alkyle ayant 1 à 4 atomes de carbone ou trifluorométhyle, ou par un groupe α-, β- ou γ-pyridyle ou par un groupe amino, ce groupe amino portant deux substituants, identiques ou différents, du groupe des substituants alkyle ayant 1 à 4 atomes de carbone, phényle ou benzyle,

$R^2$ est un reste phényle ou naphtyle, les restes mentionnés pouvant porter

jusqu'à deux substituants, identiques ou différents, fluoro, chloro, bromo, alkyle en $C_1$ à $C_6$, alkoxy en $C_1$ à $C_6$, alkylthio en $C_1$ à $C_4$, alkylsulfonyle en $C_1$ à $C_4$, trifluorométhyle, trifluorométhoxy, difluorométhoxy, nitro, cyano, (alkoxy en $C_1$ à $C_4$)carbonyle ou di(alkyle en $C_1$ à $C_4$)amino,

$R^3$ est un reste alkyle en $C_1$ à $C_6$ ou

un reste phényle ou naphtyle, les restes mentionnés pouvant être substitués par du fluor, du chlore, un radical méthyle, méthoxy ou trifluorométhyle

et

X, Ysont identiques ou différents et représentent

un groupe de formule $-CN$, $-COR^4$, $-SO_2R^4$, $-COOR^5$, $-CONR^6R^7$ ou $-SO_2NR^6R^7$, où

$R^4$ est un groupe alkyle en $C_1$ à $C_6$, phényle ou benzyle,

$R^5$ est un groupe alkyle en $C_1$ à $C_6$ ou phényle,

et

$R^6$, $R^7$ sont identiques ou différents et représentent un groupe alkyle en $C_1$ à $C_4$, phényle ou benzyle,

sous forme de leurs isomères, de leurs mélanges d'isomeres, de leurs antipodes optiques ou de leurs racémates.

3. Composés de formule générale (I) suivant la revendication 1, dans laquelle

$R^1$ est un reste hydrocarboné à chaîne droite, à chaîne ramifiée ou cyclique ayant jusqu'à 8 atomes de carbone, qui peut être interrompu dans sa chaîne par un atome d'oxygène et/ou qui peut être substitué par un radical fluoro, cyano, acétoxy, hydroxy, phényle, phénoxy, α-, β- ou γ-pyridyle ou par un groupe amino, ce groupe amino pouvant porter deux substituants identiques ou différents choisis dans le groupe des substituants alkyle ayant 1 ou 2 atomes de carbone et benzyle,

$R^2$ est un groupe phényle qui peut porter jusqu'à 2 substituants, identiques ou différents, fluoro, chloro, alkyle en $C_1$ à $C_4$, alkoxy en $C_1$ à $C_4$, trifluorométhyle ou nitro,

$R^3$ est un groupe méthyle ou phényle,

et

X, Y sont identiques ou différents et représentent
un groupe de formule –CN, –CCR⁴, $-SO_2R^4$ ou $-COOR^5$,
où
$R^4$ est un radical alkyle en $C_1$ à $C_4$, phenyle ou benzyle
et
$R^5$ est un radical alkyle en $C_1$ à $C_4$, sous forme de leurs isomères, de leurs mélanges d'isomères, de leurs antipodes optiques ou de leurs racémates.

4. Procédé de production d'esters d'acides cyclohexènecarboxyliques de formule générale (I)

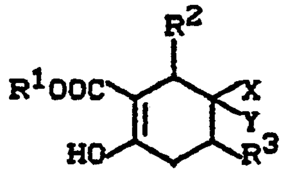

(I)

dans laquelle
$R^1$ représente un reste hydrocarboné saturé ou non saturé, à chaîne droite, à chaîne ramifiée ou cyclique ayant jusqu'à 20 atomes de carbone, qui peut être interrompu dans sa chaîne par un atome d'oxygène ou par un atome de soufre et/ou qui peut être substitué par un halogène, un radical cyano, hydroxy, acétyloxy, nitro ou par un groupe phényle, phénoxy, phénylthio ou phénylsulfonyle éventuellement substitué par un radical halogéno, cyano, dialkylamino ayant 1 ou 2 atomes de carbone par groupe alkyle, un radical alkoxy ayant 1 à 4 atomes de carbone, alkyle ayant 1 à 4 atomes de carbone, trifluorométhyle ou nitro, ou par un groupe α-, β- ou γ-pyridyle ou par un groupe amino, ce groupe amino portant 2 substituants, identiques ou différents, du groupe des substituants alkyle ayant jusqu'à 4 atomes de carbone, alkoxyalkyle ayant jusqu'à 4 atomes de carbone, phényle ou aralkyle en $C_7$ à $C_{14}$, ou bien ces substituants forment le cas échéant avec l'atome d'azote un noyau pentagonal à heptagonal qui peut comporter comme autre hétéroatome un atome d'oxygène ou de soufre ou un groupement N-phényle ou N-alkyle, ce groupe alkyle comprenant 1 à 3 atomes de carbone,
$R^2$ est un groupe aryle en $C_6$ à $C_{12}$, qui peut porter 1 à 3 substituants, identiques ou différents, halogéno, alkyle en $C_1$ à $C_8$, alkoxy en $C_1$ à $C_8$, alkylthio en $C_1$ à $C_6$, alkylsulfonyle en $C_1$ à $C_6$, trifluorométhyle, trifluorométhoxy, difluorométhoxy, trifluorométhylthio, nitro, cyano, (alkoxy en $C_1$ à $C_4$)carbonyle, di(alkyle en $C_1$ à $C_6$-amino, diméthylaminosulfonyle ou diméthylcarbamoyle,
$R^3$ est un groupe alkyle en $C_1$ à $C_6$ ou
un groupe aryle en $C_6$ à $C_{12}$ qui peut porter jusqu'à 2 substituants, identiques ou différents, halogéno, alkyle en $C_1$ à $C_4$, alkoxy en $C_1$ à $C_4$, cyano, trifluorométhyle ou trifluorométhoxy
et
X, Y sont identiques ou différents et représentent
– un groupe de formule –CN, $-COR^4$, $-SO_2R^4$, $-COOR^5$, $-CONR^6R^7$ ou $-SO_2NR^6R^7$, où
$R^4$ est un radical alkyle en $C_1$ à $C_8$, aryle en $C_6$ à $C_{12}$ ou aralkyle en $C_7$ à $C_{14}$,
$R^5$ est un radical alkyle en $C_1$ à $C_8$ ou aryle en $C_6$ à $C_{12}$ et
$R^6$, $R^7$ sont identiques ou différents et représentent un radical alkyle en $C_1$ à $C_6$, aryle en $C_6$ à $C_{12}$ ou aralkyle en $C_7$ à $C_{14}$, caractérisé en ce qu'on fait réagir des esters acétylacétiques de formule générale (II)

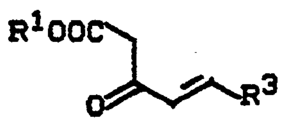

(II)

dans laquelle
$R^1$ et $R^3$ ont la définition indiquée ci-dessus, avec des éthènes de formule génèrale (III),

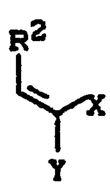

(III)

dans laquelle
$R^2$, X et Y ont la définition indiquée çi-dessus,
en présence de solvants inertes et de bases à des températures comprises entre 0°C et 100°C et on sépare éventuellement leurs formes stéréoisomériques par des procédés classiques.

5. Procédé suivant la revendication 4, caractérisé en ce qu'on utilise comme solvants inertes l'eau ou des solvants organiques tels que des alcools, des hydrocarbures halogénés, des éthers, des hydrocarbures, des amides, des acétates d'éthyle, le diméthylsulfoxyde, le sulfolane, la pyridine, la diméthylaminopyridine, la picoline, la morpholine ou la pipéridine ainsi que leurs mélanges et on utilise comme bases des hydroxydes, carbonates, alcoolates de métaux alcalins ou alcalino-terreux, l'ammoniac ou des amines organiques.

6. Composés de formule générale (I) suivant la revendication 1, destinés à être utilisés pour combattre des maladies du système circulatoire.

7. Médicaments contenant au moins un composé de formule générale (I) suivant la revendication 1.

8. Procédé de préparation de médicaments, caractérisé en ce qu'au moins un composé de formule générale (I) suivant la revendication 1 est mise sous une forme propre à être administrée, le cas échéant avec des substances auxiliaires et des excipients classiques.

9. Utilisation de composés de formule générale (I) suivant la revendication 1 dans la préparation de médicaments agissant sur la circulation.